# EUROPEAN PATENT SPECIFICATION

(11) **EP 4 279 487 B1**
(45) Date of publication and mention of the grant of the patent: **29.04.2026**
(21) Application number: 22738959.0
(22) Date of filing: 10.01.2022
(51) Int. Cl.: C07D 307/87

(54) **PURIFICATION METHOD FOR CITALOPRAM OR S-CITALOPRAM**
REINIGUNGSVERFAHREN FÜR CITALOPRAM ODER S-CITALOPRAM
PROCÉDÉ DE PURIFICATION DE CITALOPRAM OU DE S-CITALOPRAM

(30) Priority: 14.01.2021 CN 202110047911
(43) Date of publication of application: 22.11.2023
(73) Proprietor: Zhejiang Huahai Pharmaceutical Co., Ltd., Taizhou, Zhejiang 317024 (CN); Zhejiang Huahai Licheng Pharmaceutical Co., Ltd., Taizhou, Zhejiang 317024 (CN)
(72) Inventor: ZHANG, Jian, Zhejiang 317024 (CN); WANG, Jichao, Zhejiang 317024 (CN); HAN, Zheng, Zhejiang 317024 (CN); XU, Yihong, Zhejiang 317024 (CN); HU, Jiaxing, Zhejiang 317024 (CN); HUANG, Wenfeng, Zhejiang 317024 (CN); JIN, Yongjun, Zhejiang 317024 (CN)
(74) Representative: Novagraaf International SA
(86) International application number: PCT/CN2022/070997
(87) International publication number: WO 2022/152078

(56) References cited:
- EP-A1- 1 479 673
- WO-A2-01/02383
- WO-A2-02/48133
- CN-A- 1 359 382
- CN-A- 1 366 525
- CN-A- 1 688 565
- CN-A- 101 309 924
- CN-A- 101 492 436
- CN-A- 104 829 571
- NIE YANJUN, WANG WENXIN; ZHENG JING; DOU YANLI; XU YUWEN: "Study on related substances of Escitalopram Oxalate Tablets and its influencing factors", JOURNAL OF PHARMACEUTICAL RESEARCH, vol. 38, no. 1, 15 January 2019 (2019-01-15), pages 30 - 22,45, XP055950170, ISSN: 2095-5375, DOI: 10.13506/j.cnki.jpr.2019.01.008

## Description

### FIELD OF THE INVENTION

The present invention relates to the technical field of pharmaceutical chemistry, in particular to a purification method for citalopram or s-citalopram.

### BACKGROUND OF THE INVENTION

Citalopram hydrobromide is a racemic mixture, wherein the active ingredient is mainly its levoisomer.

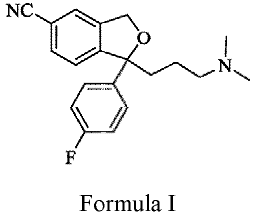

1-(3-Dimethylaminopropyl)-1-(4-fluorophenyl)-1,3-dihydroisobenzofuran-5-formaldehyde (Formula II), commonly known as aldehyde impurity, is an inherent impurity in citalopram hydrobromide and escitalopram (S-citalopram) oxalate. This impurity is similar in nature to the product and is extremely difficult to remove, and generally has a content level of about 0.1% to about 0.4%. Currently, there is no relevant literature reporting the source and removal of this impurity.

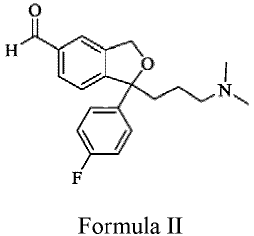

EP1479673A1 discloses a process for preparing purified citalopram or one of its salts that comprises the purification of citalopram by selective extractions of citalopram or of its impurities with organic solvents and water under specific conditions of pH and temperature.

In view of the above problems regarding the impurity, it is necessary to develop a purification method for citalopram or S-citalopram by removing the compound of Formula II.

### SUMMARY

The present invention provides a purification method for citalopram or S-citalopram or a salt thereof, comprising the following steps:
(a) treating a solution consisting of citalopram and a water-immiscible organic solvent with a washing solution, and carrying out a separation to obtain an organic layer containing citalopram; or, treating a solution consisting of S-citalopram and a water-immiscible organic solvent with a washing solution, and carrying out a separation to obtain an organic layer containing S-citalopram; and
(b) further separating the organic layer obtained in step (a) to obtain citalopram or S-citalopram; or further adding an acid to form a salt and carrying out a separation to obtain an acid salt of citalopram or an acid salt of S-citalopram;

wherein, the washing solution as defined in step (a) is an aqueous solution comprising a washing agent, and the washing agent is selected from the group consisting of sodium dithionite, sodium thiosulfate, and sodium sulfite; and
wherein, citalopram or S-citalopram or the salt thereof is purified by removing 1-(3-Dimethylaminopropyl)-1-(4-fluorophenyl)-1,3-dihydroisobenzofuran-5-formaldehyd (Formula II).

In a preferred embodiment of the present invention:
the water-immiscible organic solvent as defined in step (a) is selected from the group consisting of toluene, ethyl acetate, methyl isobutyl ketone, and chlorobenzene, or any combinations thereof.

The solution consisting of citalopram and the water-immiscible organic solvent or the solution consisting of S-citalopram and the water-immiscible organic solvent as defined in step (a) is obtained by forming a free base from an acid salt of citalopram or acid salt of S-citalopram, which is obtained by the following process: mixing the acid salt of citalopram or the acid salt of S-citalopram, water and the water-immiscible organic solvent, adding a base to obtain a free base, and carrying out a separation to obtain an organic layer; or the solution can also be derived from a reaction solution in a synthesis process of citalopram or S-citalopram, and the reaction solution can be prepared with reference to technical solutions in the prior art. Preferably, the solution is obtained by forming a free base from citalopram hydrobromide or S-citalopram oxalate.

The washing agent in the washing solution as defined in step (a) is sodium dithionite.

A molar ratio of the washing agent and citalopram or S-citalopram ranges from 0.05 to 1.5.

A mass ratio of the washing agent and water in the washing solution as defined in step (a) ranges from 1% to 30%.

In one embodiment, an inorganic base is added to the washing solution as defined in step (a), and the inorganic base is selected from the group consisting of sodium carbonate, sodium bicarbonate, potassium carbonate, potassium bicarbonate, sodium hydroxide and potassium hydroxide, or any combinations thereof; preferably the inorganic base is sodium bicarbonate.

A mass ratio of the inorganic base and the washing agent ranges from 5% to 30%.

The acid as defined in step (b) is selected from the group consisting of hydrochloric acid, hydrobromic acid, phosphoric acid, oxalic acid, fumaric acid, acetic acid, p-toluenesulfonic acid and methanesulfonic acid; preferably the acid is hydrobromic acid or oxalic acid.

In the present invention, the washing solution as defined in step (a) is an aqueous solution containing washing agent. The preparation process is not limited in the present application, as long as it can achieve the purpose of the present invention. Exemplarily, it is prepared in accordance with the following steps: adding the washing agent and water, and optionally the inorganic base, to a container successively, and stirring the mixture until dissolved and clarified to obtain the washing solution.

The process of stirring the washing solution as defined in step (a) is not limited in the present invention, as long as it can achieve the purpose of the present invention. Exemplarily, it can be achieved by stirring the mixture at a temperature of 40°C to 45°C for 60 min to 90 min.

The process of further separating the organic layer to obtain citalopram or S-citalopram as defined in step (b) is not limited in the present invention, as long as it can achieve the purpose of the invention. Exemplarily, the separation is performed by concentrating the organic layer to dryness under reduced pressure.

In the present invention, the process of further adding acid to form a salt as defined in step (b) is performed by dissolving the organic layer that has been concentrated to dryness in an organic solvent under a controlled temperature. In the present invention, the organic solvent is selected from the group consisting of toluene, ethyl acetate, methanol, methyl isobutyl ketone and anhydrous ethanol, or any combinations thereof; and the temperature is controlled in the range of 40°C to 60°C. Exemplarily, the organic layer is concentrated to dryness under reduced pressure, added to ethyl acetate and dissolved, and the temperature is controlled at 50°C to 60°C.

The process of further adding acid to form a salt as defined in step (b) is performed by dissolving the organic layer and adding an acid dropwise to adjust pH=4±0.5 so as form the salt, and the acid salt of citalopram or acid salt of S-citalopram is obtained through separation. In the present invention, the acid is selected from the group consisting of hydrochloric acid, hydrobromic acid, phosphoric acid, oxalic acid, fumaric acid, acetic acid, p-toluenesulfonic acid, and methanesulfonic acid. Exemplarily, hydrobromic acid is added dropwise to adjust pH=4±0.5 to form a salt, and then the resultant is refluxed for 2 hours, cooled down to crystallize a crystal, which is then filtered and dried to obtain citalopram hydrobromide.

The purification method for citalopram or S-citalopram provided by the present invention is simple in operation and high in impurity removal rate, cheap and easily available in raw and auxiliary materials, and mild in conditions, which is conducive to the simple and efficient purification of citalopram or S-citalopram.

### DETAILED DESCRIPTION

The invention is further described in detail by specific examples below. All raw materials used in the examples are commercially available.

### Example 1

In a beaker, 2.5 g of sodium dithionite and 50 mL of drinking water were added successively, and the mixture was stirred until dissolved and clarified to obtain a washing solution.

In a three-necked flask, 25 g of citalopram hydrobromide (containing 0.364% aldehyde impurity), 200 mL of toluene and 100 mL of drinking water were added successively. The temperature was raised to 45°C. Ion-exchange membrane liquid caustic soda was slowly added dropwise to adjust pH of the aqueous solution to 12. The resultant was stirred until dissolved and clarified, and allowed to stand and layer. An organic layer was obtained by separation, and was added with the washing solution. Under a controlled temperature of 40°C to 45°C, the mixture was stirred for 60 min and allowed to stand and layer. An organic layer was obtained by separation, and was concentrated to dryness under reduced pressure, which was then dissolved by 120 mL of ethyl acetate. Under a controlled temperature of 50°C to 60°C, hydrobromic acid was added dropwise to adjust pH=4±0.5 to form a salt, and the resultant was then refluxed for 2 hours and cooled down to crystallize a crystal, which was then filtered and dried to obtain citalopram hydrobromide. Yield: 90%; purity: 99.7%; aldehyde impurity: 0.04%; impurity removal rate: 89.0%.

### Example 2

In a beaker, 2.5 g of sodium dithionite, 50 mL of drinking water and 0.25 g of sodium bicarbonate were added successively, and the mixture was stirred until dissolved and clarified to obtain a washing solution.

In a three-necked flask, 25 g of citalopram hydrobromide (containing 0.470% aldehyde impurity), 200 mL of toluene and 100 mL of drinking water were added successively. The temperature was raised to 45°C. Ion-exchange membrane liquid caustic soda was slowly added dropwise to adjust pH of the aqueous solution to 12. The resultant was stirred until dissolved and clarified, and allowed to stand and layer. An organic layer was obtained by separation, and was added with the washing solution. Under a controlled temperature of 40°C to 45°C, the mixture was stirred for 60 min and allowed to stand and layer. An organic layer was obtained by separation, and was concentrated to dryness under reduced pressure, which was then dissolved by 120 mL of ethyl acetate. Under a controlled temperature of 50°C to 60°C, hydrobromic acid was added dropwise to adjust pH=4±0.5 to form a salt, and the resultant was then refluxed for 2 hours and cooled down to crystallize a crystal, which was then filtered and dried to obtain citalopram hydrobromide. Yield: 91%; purity: 99.8%; aldehyde impurity: 0.029%; impurity removal rate: 93.8%.

### Example 3

In a beaker, 0.5 g of sodium dithionite, 50 mL of drinking water and 0.05 g of sodium bicarbonate were added successively, and the mixture was stirred until dissolved and clarified to obtain a washing solution.

In a three-necked flask, 25 g of S-citalopram oxalate (containing 0.364% aldehyde impurity), 200 mL of methyl isobutyl ketone and 100 mL of drinking water were added successively. The temperature was raised to 45°C. 30% potassium hydroxide solution was slowly added dropwise to adjust pH of the aqueous solution to 12. The resultant was stirred until dissolved and clarified, and allowed to stand and layer. An organic layer was obtained by separation, and was added with the washing solution. Under a controlled temperature of 40°C to 45°C, the mixture was stirred for 60 min and allowed to stand and layer. An organic layer was obtained by separation, and was concentrated to dryness under reduced pressure, which was then added with 48 mL of anhydrous ethanol. The resultant was stirred until dissolved and clarified, and under a controlled temperature of 40°C to 50°C, 9.1g of oxalic acid was added. The mixture was stirred for 2 hours and cooled down to crystallize a crystal, which was then filtered and dried to obtain S-citalopram oxalate. Yield: 88%; purity: 99.9%; aldehyde impurity: 0.033%; impurity removal rate: 90.9%.

### Example 4

In a beaker, 2.3 g of sodium thiosulfate, 50 mL of drinking water and 0.23 g of sodium carbonate were added successively, and the mixture was stirred until dissolved and clarified to obtain a washing solution.

In a three-necked flask, 25 g of citalopram hydrobromide (containing 0.470% aldehyde impurity), 200 mL of ethyl acetate and 100 mL of drinking water were added successively. The temperature was raised to 45°C. Ion-exchange membrane liquid caustic soda was slowly added dropwise to adjust pH of the aqueous solution to 12. The resultant was stirred until dissolved and clarified, and allowed to stand and layer. An organic layer was obtained by separation, and was added with the washing solution. Under a controlled temperature of 40°C to 45°C, the mixture was stirred for 60 min and allowed to stand and layer. An organic layer was obtained by separation, and was concentrated to dryness under reduced pressure, which was then dissolved by 120 mL of ethyl acetate. Under a controlled temperature of 50°C to 60°C, hydrobromic acid was added dropwise to adjust pH=4±0.5 to form a salt, and the resultant was refluxed for 2 hours and cooled down to crystallize a crystal, which was then filtered and dried to obtain citalopram hydrobromide. Yield: 92%; purity: 99.8%; aldehyde impurity: 0.06%; impurity removal rate: 87.2%.

### Example 5

In a beaker, 1.8 g of sodium sulfite, 50 mL of drinking water and 0.54 g of potassium bicarbonate were added successively, and the mixture was stirred until dissolved and clarified to obtain a washing solution.

In a three-necked flask, 25 g of citalopram hydrobromide (containing 0.303% aldehyde impurity), 200 mL of chlorobenzene and 100 mL of drinking water were added successively. The temperature was raised to 45°C. Ion-exchange membrane liquid caustic soda was slowly added dropwise to adjust pH of the aqueous solution to 12. The resultant was stirred until dissolved and clarified, and allowed to stand and layer. An organic layer was obtained by separation, and was added with the washing solution. Under a controlled temperature of 40°C to 45°C, the mixture was stirred for 60 min and allowed to stand and layer. An organic layer was obtained by separation, and was concentrated to dryness under reduced pressure, which was then dissolved by 120 mL of ethyl acetate. Under a controlled temperature of 50°C to 60°C, hydrobromic acid was added dropwise to adjust pH=4±0.5 to form a salt, and the resultant was refluxed for 2 hours and cooled down to crystallize a crystal, which was then filtered and dried to obtain citalopram hydrobromide. Yield: 91%; purity: 99.7%; aldehyde impurity: 0.023%; impurity removal rate: 92.4%.

### Example 6

In a beaker, 15 g of sodium dithionite, 50 mL of drinking water and 0.75 g of potassium carbonate were added successively, and the mixture was stirred until dissolved and clarified to obtain a washing solution.

In a three-necked flask, 25 g of citalopram hydrobromide (containing 0.303% aldehyde impurity), 200 mL of toluene and 100 mL of drinking water were added successively. The temperature was raised to 45°C. Ion-exchange membrane liquid caustic soda was slowly added dropwise to adjust pH of the aqueous solution to 12. The resultant was stirred until dissolved and clarified, and allowed to stand and layer. An organic layer was obtained by separation, and was added with the washing solution. Under a controlled temperature of 40°C to 45°C, the mixture was stirred for 60 min and allowed to stand and layer. An organic layer was obtained by separation, and was concentrated to dryness under reduced pressure, which was then dissolved by 120 mL of ethyl acetate. Under a controlled temperature of 50°C to 60°C, hydrobromic acid was added dropwise to adjust pH=4±0.5 to form a salt, and the resultant was refluxed for 2 hours and cooled down to crystallize a crystal, which was then filtered and dried to obtain citalopram hydrobromide. Yield: 90%; purity: 99.8%; aldehyde impurity: 0.041%; impurity removal rate: 86.5%.

### Example 7

In a beaker, 2.5 g of sodium dithionite, 50 mL of drinking water and 0.25 g of sodium hydroxide were added successively, and the mixture was stirred until dissolved and clarified to obtain a washing solution.

In a three-necked flask, 25 g of citalopram hydrobromide (containing 0.522% aldehyde impurity), 200 mL of methyl isobutyl ketone and 100 mL of drinking water were added successively. The temperature was raised to 45°C. Ion-exchange membrane liquid caustic soda was slowly added dropwise to adjust pH of the aqueous solution to 12. The resultant was stirred until dissolved and clarified, and allowed to stand and layer. An organic layer was obtained by separation, and was added with the washing solution. Under a controlled temperature of 40°C to 45°C, the mixture was stirred for 60 min and allowed to stand and layer. An organic layer was obtained by separation, and was concentrated to dryness under reduced pressure, which was then dissolved by 120 mL of ethyl acetate. Under a controlled temperature of 50°C to 60°C, hydrobromic acid was added dropwise to adjust pH=4±0.5 to form a salt, and the resultant was refluxed for 2 hours and cooled down to crystallize a crystal, which was then filtered and dried to obtain citalopram hydrobromide. Yield: 93%; purity: 99.8%; aldehyde impurity: 0.04%; impurity removal rate: 92.3%.

The above mentioned examples are only preferred examples of the present invention, and are not used to limit the invention.

## Claims

1. A purification method for citalopram or S-citalopram or a salt thereof, comprising the following steps:
(a) treating a solution consisting of citalopram and a water-immiscible organic solvent with a washing solution, and carrying out a separation to obtain an organic layer containing citalopram; or, treating a solution consisting of S-citalopram and a water-immiscible organic solvent with a washing solution, and carrying out a separation to obtain an organic layer containing S-citalopram; and
(b) further separating the organic layer obtained in step (a) to obtain citalopram or S-citalopram; or further adding an acid to form a salt and carrying out a separation to obtain an acid salt of citalopram or an acid salt of S-citalopram;
wherein, the washing solution in step (a) is an aqueous solution comprising a washing agent, and the washing agent is selected from the group consisting of sodium dithionite, sodium thiosulfate, and sodium sulfite; and
wherein, citalopram or S-citalopram or the salt thereof is purified by removing 1-(3-dimethylaminopropyl)-1-(4-fluorophenyl)-1,3-dihydroisobenzofuran-5-formaldehyde.

2. The purification method according to claim 1, wherein the water-immiscible organic solvent in step (a) is selected from the group consisting of toluene, ethyl acetate, methyl isobutyl ketone, and chlorobenzene, or any combinations thereof.

3. The purification method according to claim 1, wherein the washing agent is sodium dithionite.

4. The purification method according to claim 1, wherein a mass ratio of the washing agent and water in the washing solution of step (a) ranges from 1% to 30%.

5. The purification method according to claim 1 or 3, wherein a molar ratio of the washing agent and citalopram or S-citalopram ranges from 0.05 to 1.5.

6. The purification method according to claim 1, wherein an inorganic base is added to the washing solution of step (a).

7. The purification method according to claim 6, wherein the inorganic base is selected from the group consisting of sodium carbonate, sodium bicarbonate, potassium carbonate, potassium bicarbonate, sodium hydroxide and potassium hydroxide, or any combinations thereof; preferably the inorganic base is sodium bicarbonate.

8. The purification method according to claim 6, wherein a mass ratio of the inorganic base and the washing agent ranges from 5% to 30%.

9. The method according to claim 1, wherein the acid in step (b) is selected from the group consisting of hydrochloric acid, hydrobromic acid, phosphoric acid, oxalic acid, fumaric acid, acetic acid, p-toluenesulfonic acid and methanesulfonic acid, preferably the acid is hydrobromic acid or oxalic acid.

10. The purification method according to claim 1, wherein the solution consisting of citalopram and the water-immiscible organic solvent or the solution consisting of S-citalopram and the water-immiscible organic solvent in step (a) is obtained by: mixing citalopram hydrobromide or S-citalopram oxalate, water, and the water-immiscible organic solvent, adding a base to obtain a free base of citalopram or S-citalopram, and carrying out a separation to obtain an organic layer.

## Patentansprüche

1. Reinigungsverfahren für Citalopram oder S-Citalopram oder ein Salz davon, umfassend die folgenden Schritte:
(a) Behandeln einer Lösung bestehend aus Citalopram und einem mit Wasser nicht mischbaren organischen Lösungsmittel mit einer Waschlösung und Durchführen einer Trennung, um eine Citalopram enthaltende organische Schicht zu erhalten; oder Behandeln einer Lösung bestehend aus S-Citalopram und einem mit Wasser nicht mischbaren organischen Lösungsmittel mit einer Waschlösung und Durchführen einer Trennung, um eine S-Citalopram enthaltende organische Schicht zu erhalten; und
(b) weiteres Trennen der in Schritt (a) erhaltenen organischen Schicht, um Citalopram oder S-Citalopram zu erhalten; oder weiteres Hinzufügen einer Säure, um ein Salz zu bilden, und Durchführen einer Trennung, um ein Säuresalz von Citalopram oder ein Säuresalz von S-Citalopram zu erhalten;
wobei die Waschlösung in Schritt (a) eine wässrige Lösung ist, die ein Waschmittel umfasst, und das Waschmittel ausgewählt ist aus der Gruppe bestehend aus Natriumdithionit, Natriumthiosulfat und Natriumsulfit; und
wobei Citalopram oder S-Citalopram oder das Salz davon durch Entfernen von 1-(3-Dimethylaminopropyl)-1-(4-fluorphenyl)-1,3-dihydroisobenzofuran-5-formaldehyd gereinigt wird.

2. Reinigungsverfahren nach Anspruch 1, wobei das mit Wasser nicht mischbare organische Lösungsmittel in Schritt (a) ausgewählt ist aus der Gruppe bestehend aus Toluol, Ethylacetat, Methylisobutylketon und Chlorbenzol oder einer beliebigen Kombination davon.

3. Reinigungsverfahren nach Anspruch 1, wobei das Waschmittel Natriumdithionit ist.

4. Reinigungsverfahren nach Anspruch 1, wobei ein Massenverhältnis des Waschmittels und Wasser in der Waschlösung von Schritt (a) in einem Bereich von 1 % bis 30 % liegt.

5. Reinigungsverfahren nach Anspruch 1 oder 3, wobei ein Molverhältnis des Waschmittels und Citalopram oder S-Citalopram in einem Bereich von 0,05 bis 1,5 liegt.

6. Reinigungsverfahren nach Anspruch 1, wobei der Waschlösung von Schritt (a) eine anorganische Base hinzugefügt wird.

7. Reinigungsverfahren nach Anspruch 6, wobei die anorganische Base ausgewählt ist aus der Gruppe bestehend aus Natriumcarbonat, Natriumhydrogencarbonat, Kaliumcarbonat, Kaliumhydrogencarbonat, Natriumhydroxid und Kaliumhydroxid oder beliebigen Kombinationen davon; wobei die anorganische Base vorzugsweise Natriumhydrogencarbonat ist.

8. Reinigungsverfahren nach Anspruch 6, wobei ein Massenverhältnis der anorganischen Base und des Waschmittels in einem Bereich von 5 % bis 30 % liegt.

9. Verfahren nach Anspruch 1, wobei die Säure in Schritt (b) ausgewählt ist aus der Gruppe bestehend aus Chlorwasserstoffsäure, Bromwasserstoffsäure, Phosphorsäure, Oxalsäure, Fumarsäure, Essigsäure, p-Toluolsulfonsäure und Methansulfonsäure, wobei die Säure vorzugsweise Bromwasserstoffsäure oder Oxalsäure ist.

10. Reinigungsverfahren nach Anspruch 1, wobei die Lösung bestehend aus Citalopram und dem mit Wasser nicht mischbaren organischen Lösungsmittel oder die Lösung bestehend aus S-Citalopram und dem mit Wasser nicht mischbaren organischen Lösungsmittel in Schritt (a) durch Folgendes erhalten wird: Mischen von Citalopramhydrobromid oder S-Citalopramoxalat, Wasser und dem mit Wasser nicht mischbaren organischen Lösungsmittel, Hinzufügen einer Base, um eine freie Base von Citalopram oder S-Citalopram zu erhalten und Durchführen einer Trennung, um eine organische Schicht zu erhalten.

## Revendications

1. Procédé de purification de citalopram ou de S-citalopram ou d'un sel de ceux-ci, comprenant les étapes suivantes :
(a) le traitement d'une solution composée de citalopram et d'un solvant organique non miscible à l'eau avec une solution de lavage, et la réalisation d'une séparation pour obtenir une phase organique contenant du citalopram ; ou, le traitement d'une solution composée de S-citalopram et d'un solvant organique non miscible à l'eau avec une solution de lavage, et la réalisation d'une séparation pour obtenir une phase organique contenant du S-citalopram ; et
(b) une nouvelle séparation de la phase organique obtenue à l'étape (a) pour obtenir du citalopram ou du S-citalopram ; ou l'ajout supplémentaire d'un acide pour former un sel puis la réalisation d'une séparation pour obtenir un sel acide de citalopram ou un sel acide de S-citalopram ;
dans lequel la solution de lavage de l'étape (a) est une solution aqueuse comprenant un agent de lavage, et l'agent de lavage est choisi parmi le groupe constitué par dithionite de sodium, thiosulfate de sodium et sulfite de sodium ; et
dans lequel le citalopram ou le S-citalopram ou le sel de ceux-ci est purifié par élimination du 1-(3-diméthylaminopropyl)-1-(4-fluorophényl)-1,3-dihydroisobenzofuran-5-formaldéhyde.

2. Procédé de purification selon la revendication 1, dans lequel le solvant organique non miscible à l'eau de l'étape (a) est choisi parmi le groupe constitué par toluène, acétate d'éthyle, méthylisobutylcétone et chlorobenzène, ou toute combinaison de ceux-ci.

3. Procédé de purification selon la revendication 1, dans lequel l'agent de lavage est le dithionite de sodium.

4. Procédé de purification selon la revendication 1, dans lequel le rapport massique entre l'agent de lavage et l'eau dans la solution de lavage de l'étape (a) varie de 1 % à 30 %.

5. Procédé de purification selon la revendication 1 ou 3, dans lequel le rapport molaire entre l'agent de lavage et le citalopram ou le S-citalopram varie de 0,05 à 1,5.

6. Procédé de purification selon la revendication 1, dans lequel une base inorganique est ajoutée à la solution de lavage de l'étape (a).

7. Procédé de purification selon la revendication 6, dans lequel la base inorganique est choisie parmi le groupe constitué par carbonate de sodium, bicarbonate de sodium, carbonate de potassium, bicarbonate de potassium, hydroxyde de sodium et hydroxyde de potassium, ou toute combinaison de ceux-ci ; de préférence, la base inorganique est le bicarbonate de sodium.

8. Procédé de purification selon la revendication 6, dans lequel le rapport massique entre la base inorganique et l'agent de lavage varie de 5 % à 30 %.

9. Procédé selon la revendication 1, dans lequel l'acide de l'étape (b) est choisi parmi le groupe constitué par acide chlorhydrique, acide bromhydrique, acide phosphorique, acide oxalique, acide fumarique, acide acétique, acide p-toluènesulfonique et acide méthanesulfonique, de préférence l'acide est l'acide bromhydrique ou l'acide oxalique.

10. Procédé de purification selon la revendication 1, dans lequel la solution composée de citalopram et du solvant organique non miscible à l'eau ou la solution composée de S-citalopram et du solvant organique non miscible à l'eau de l'étape (a) est obtenue par : le mélange d'hydrobromure de citalopram ou d'oxalate de S-citalopram, d'eau et du solvant organique non miscible à l'eau, l'ajout d'une base pour obtenir une base libre de citalopram ou de S-citalopram, et la réalisation d'une séparation pour obtenir une phase organique.
